# EUROPEAN PATENT SPECIFICATION

(11) **EP 3 983 519 B1**
(45) Date of publication and mention of the grant of the patent: **07.08.2024**
(21) Application number: 20733702.3
(22) Date of filing: 09.06.2020
(51) Int. Cl.: C12M 1/00

(54) **BIOPROCESSING SYSTEM**
BIOVERARBEITUNGSSYSTEM
SYSTÈME DE BIOTRAITEMENT

(30) Priority: 12.06.2019 IN 201911023338
(43) Date of publication of application: 20.04.2022
(60) Divisional application: 24150446.3
(73) Proprietor: Global Life Sciences Solutions USA LLC, Marlborough, MA 01752 (US)
(72) Inventor: BECKER, Timothy, J, Westborough, Massachusetts 01581-1019 (US); DASH, Manas, Marlborough, Massachusetts 01752 (US); STANKOWSKI, Ralph, Marlborough, Massachusetts 01752 (US); TUOHEY, Colin, Marlborough, MA 01752 (US); PARZIALE, Michelle Ann, Marlborough, Massachusetts 01752 (US); STEGNER, Elizabeth, Marlborough, Massachusetts 01752 (US); KILAR, Alexander, Mark, Marlborough, Massachusetts 01752 (US); MURUGESAN, Kandakumar, Karnataka 560 066 (IN); BAGIYANATHAN, Prasad, Karnataka 560 066 (IN); CHERUNNI, Sairam, Karnataka 560 066 (IN); CHANDRAMOULI, Nikhil, Vagga, Karnataka 560 066 (IN)
(74) Representative: Bedford, Grant Richard
(86) International application number: PCT/EP2020/065935
(87) International publication number: WO 2020/249549

(56) References cited:
- US-A1- 2012 175 012
- US-A1- 2012 284 991
- US-A1- 2015 056 694
- US-A1- 2015 190 764
- US-A1- 2016 304 824
- US-B2- 8 123 199

## Description

### TECHNICAL FIELD

Embodiments of the invention relate generally to bioprocessing systems.

### DISCUSSION OF ART

A variety of vessels, devices, components and unit operations are known for carrying out biochemical and/or biological processes and/or manipulating liquids and other products of such processes. In order to avoid the time, expense, and difficulties associated with sterilizing the vessels used in biopharmaceutical manufacturing processes, single-use or disposable bioreactor bags and single-use mixer bags are used as such vessels. For instance, biological materials (e.g., animal and plant cells) including, for example, mammalian, plant or insect cells and microbial cultures can be processed using disposable or single-use mixers and bioreactors.

Increasingly, in the biopharmaceutical industry, single use or disposable containers are used. See, for example, US 2015/190764 A1 and US 8,123,199 B2. Such containers can be flexible or collapsible plastic bags that are supported by an outer rigid structure such as a stainless steel shell or vessel. Use of sterilized disposable bags eliminates time-consuming step of cleaning of the vessel and reduces the chance of contamination. The bag may be positioned within the rigid vessel and filled with the desired fluid for mixing. Depending on the fluid being processed, the system may include a number of fluid lines and different sensors, probes and ports coupled with the bag for monitoring, analytics, sampling, and fluid transfer. For example, a plurality of ports may typically be located at the front of the bag and accessible through an opening in the sidewall of the vessel, which provide connection points for sensors, probes and/or fluid sampling lines. In addition, a harvest port or drain line fitting is typically located at the bottom of the disposable bag and is configured for insertion through an opening in the bottom of the vessel, allowing for a harvest line to be connected to the bag for harvesting and draining of the bag after the bioprocess is complete.

Typically, an agitator assembly disposed within the bag is used to mix the fluid. Existing agitators are either top-driven (having a shaft that extends downwardly into the bag, on which one or more impellers are mounted) or bottom-driven (having an impeller disposed in the bottom of the bag that is driven by a magnetic drive system or motor positioned outside the bag and/or vessel). Most magnetic agitator systems include a rotating magnetic drive head outside of the bag and a rotating magnetic agitator (also referred to in this context as the "impeller") within the bag. The movement of the magnetic drive head enables torque transfer and thus rotation of the magnetic agitator allowing the agitator to mix a fluid within the vessel. Magnetic coupling of the agitator inside the bag, to a drive system or motor external to the bag and/or bioreactor vessel, can eliminate contamination issues, allow for a completely enclosed system, and prevent leakage. Because there is no need to have a drive shaft penetrate the bioreactor vessel wall to mechanically spin the agitator, magnetically coupled systems can also eliminate the need for having seals between the drive shaft and the vessel.

Installation and setup of the flexible bioprocessing bag within the bioreactor vessel, along with the associated tubing, filter heaters, impeller and other components can be a labor intensive and time-consuming process. For example, existing bioreactor vessels may present accessibility issues, making it difficult to align and properly seat the impeller with the bioreactor vessel base. Multiple operators and ladders may also be needed, especially for the installation of tubing and filter heaters, which are located at the top of the vessel. Moreover, lack of tubing support for the various tubes connected to the flexible bag can lead to a cluttered array of tubes around the bioreactor vessel. In addition to the above, with existing systems, inflation and deflation of the flexible bioprocessing bag consumable can also a time-consuming process, taking between 10 minutes and almost an hour.

In addition to difficulties installing the flexible bioprocessing bag and other components at the top of the bioreactor vessel, properly seating the impeller base plate of the flexible bioprocessing bag on the bottom of the bioreactor vessel during installation of the flexible bag may also present challenges. In particular, with existing systems, there is no feedback mechanism, other than visual inspection, to indicate that the impeller base plate of the flexible bag is properly seated within the recess in the bottom of the bioreactor vessel. Even when a visual inspection reveals that the base plate is properly seated, movement of the base plate before mating of the agitator and magnetic drive assembly beneath the vessel is possible.

In view of the above, there is a need for a bioprocessing system that is ergonomically efficient, facilitates installation and setup, and/or assists in the inflation and deflation of the flexible bioprocessing bag.

### BRIEF DESCRIPTION

Hence, the present invention, as defined by the appended claims, is provided.

### DRAWINGS

The present invention will be better understood from reading the following description of non-limiting embodiments, with reference to the attached drawings, wherein below:
FIG. 1 is a perspective view of a bioreactor system for use with embodiments of the invention.
FIG. 2 is another perspective view of the bioreactor system of FIG. 1, illustrating an access door in a closed position.
FIG. 3 is another perspective view of the bioreactor system of FIG. 1, illustrating the access door in an open position.
FIG. 4 is a perspective view of a bioreactor system also for use with embodiments of the invention.
FIG. 5 is perspective view of the bioreactor system of FIG. 4, illustrating an access position of a tubing and component management apparatus.
FIG. 6 is a perspective view of the bioreactor system of FIG. 4, illustrating a loading position of the tubing and component management apparatus.
FIG. 7 is a perspective view of the bioreactor system of FIG. 4, illustrating consumable components installed on the tubing and component management apparatus.
FIG. 8 is a perspective view of the bioreactor system of FIG. 4, illustrating a ready position of the tubing and component management apparatus.
FIG. 9 is a perspective view of the bioreactor system of FIG. 4, illustrating an operational position of the tubing and component management apparatus.
FIG. 10 is a perspective view of a tubing and component management apparatus, for use with embodiments of the invention.
FIG. 11 is an enlarged, detail view of the tubing and component management apparatus of FIG. 10.
FIG. 12 is another enlarged, detail view of the tubing and component management apparatus of FIG. 10.
FIG. 13 is yet another enlarged, detail view of the tubing and component management apparatus of FIG. 10.
FIG. 14 is a top plan view of a bioreactor vessel with which the tubing and component management apparatus of FIG. 10 may be utilized.
FIG. 15 is perspective view of a bioreactor system for use with embodiments of the invention.
FIG. 16 is a perspective view of the bioreactor system of FIG. 15, illustrating a loading position of the tubing and component management apparatus.
FIG. 17 is a perspective view of the bioreactor system of FIG. 15, illustrating consumable components installed on the tubing and component management apparatus.
FIG. 18 is a perspective view of the bioreactor system of FIG. 15, illustrating a ready position of the tubing and component management apparatus.
FIG. 19 is a perspective view of the bioreactor system of FIG. 15, illustrating an operational position of the tubing and component management apparatus.
FIG. 20 is a perspective view of a base plate of a flexible bioprocessing bag, for use with a bioreactor system, according to an embodiment of the invention.
FIG. 21 is a top perspective view of a locking system for a base plate of a bioreactor system, according to another embodiment of the invention.
FIG. 22 is a bottom perspective view of a locking system of FIG. 21.
FIG. 23 is a side elevational view of the locking system of FIG. 21.
FIG. 24 is a cross-sectional, perspective view of a base plate locked in position within a bioreactor vessel using the locking system of FIG. 21.
FIG. 25 is a side cross-sectional view of the base plate locked in position within a bioreactor vessel using the locking system of FIG. 21.
FIG. 26 is a top perspective view of a locking and indicator system for a base plate of a bioreactor system, according to another embodiment of the invention.
FIG. 27 is a bottom perspective view of the locking and indicator system of FIG. 26.
FIG. 28 is a side view of the locking and indicator system of FIG. 26 when the baseplate is improperly positioned.
FIG. 29 is a side view of the locking and indicator system of FIG. 26 when the baseplate is properly positioned.

### DETAILED DESCRIPTION

Reference will be made below in detail to exemplary embodiments of the invention, examples of which are illustrated in the accompanying drawings. Wherever possible, the same reference characters used throughout the drawings refer to the same or like parts.

As used herein, the term "flexible" or "collapsible" refers to a structure or material that is pliable, or capable of being bent without breaking, and may also refer to a material that is compressible or expandable. An example of a flexible structure is a bag formed of polyethylene film. The terms "rigid" and "semi-rigid" are used herein interchangeably to describe structures that are "non-collapsible," that is to say structures that do not fold, collapse, or otherwise deform under normal forces to substantially reduce their elongate dimension. Depending on the context, "semi-rigid" can also denote a structure that is more flexible than a "rigid" element, e.g., a bendable tube or conduit, but still one that does not collapse longitudinally under normal conditions and forces.

A "vessel," as the term is used herein, means a flexible bag, a flexible container, a semi-rigid container, a rigid container, or a flexible or semi-rigid tubing, as the case may be. The term "vessel" as used herein is intended to encompass bioreactor vessels having a wall or a portion of a wall that is flexible or semi-rigid, single use flexible bags, as well as other containers or conduits commonly used in biological or biochemical processing, including, for example, cell culture/purification systems, mixing systems, media/buffer preparation systems, and filtration/purification systems, e.g., chromatography and tangential flow filter systems, and their associated flow paths. As used herein, the term "bag" means a flexible or semi-rigid container or vessel used, for example, as a bioreactor or mixer for the contents within. As used herein, "consumable" or "consumable component" means devices or components that are intended to be replaced regularly due to wear or use.

Embodiments of the invention provide bioprocessing systems and, in particular, tubing and components management systems and devices for a bioreactor system. In an embodiment, a bioreactor vessel includes a bottom, a peripheral sidewall, the bottom and the peripheral sidewall defining an interior space for receiving a flexible bioprocessing bag, a recess in the bottom for receiving a base plate of the flexible bioprocessing bag, and a locking mechanism configured to retain the base plate in the recess.

Further embodiments of the invention provide bioprocessing systems and, in particular, tubing and components management systems and devices for a bioreactor system. In an embodiment, a bioreactor vessel includes a bottom, a peripheral sidewall, the bottom and the peripheral sidewall defining an interior space for receiving a flexible bioprocessing bag, a recess in the bottom for receiving a base plate of the flexible bioprocessing bag, a locking mechanism configured to retain the base plate in the recess, and an indicator mechanism configured to indicate when the base plate is properly positioned within the recess.

Embodiments of the invention provide bioprocessing systems and, in particular, tubing and components management systems and devices for a bioreactor system. In an embodiment, a bioprocessing system includes a vessel defining an interior space for receiving a flexible bioprocessing bag, the vessel having an access door in a sidewall of the vessel and providing access to the interior space, and a tubing and component management apparatus mounted to the sidewall of the vessel and having a mounting frame for mounting of at least one consumable component of the bioprocessing system. The mounting frame is moveable vertically into and out of the interior space.

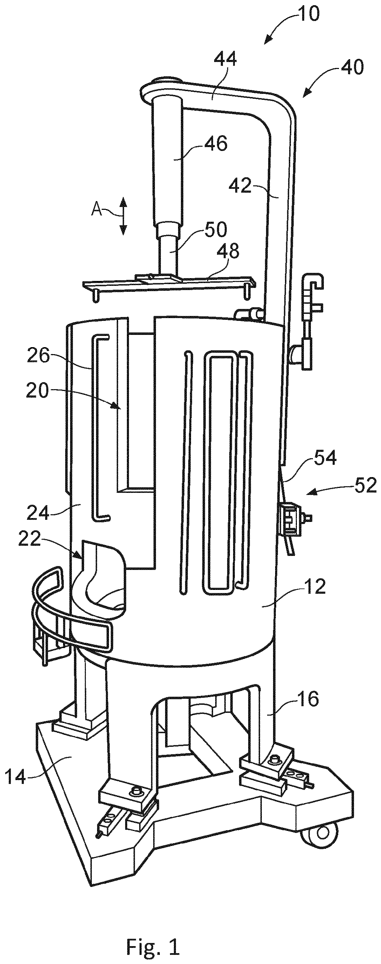

With reference to FIGS. 1-3, a bioreactor system 10 is illustrated. The bioreactor system 10 includes a generally rigid bioreactor vessel or support structure 12 mounted atop a base 14 having a plurality of legs 16. The vessel 12 may be formed, for example, from stainless steel, polymers, composites, glass, or other metals, and may be cylindrical in shape, although other shapes may also be utilized without departing from the broader aspects of the invention. The vessel 12 can be any shape or size as long as it is capable of supporting a single-use, flexible bioreactor bag in an interior space 18 thereof. For example, according to one embodiment of the invention the vessel 12 is capable of accepting and supporting a 10L-2000L flexible or collapsible bioprocess bag.

The vessel 12 may include one or more sight windows 20, which allows an operator to view a fluid level within the flexible bag positioned within the interior space 18, as well as a window 22 positioned at a lower area of the vessel 12. The window 22 allows access to the interior of the vessel 12 for insertion and positioning of various sensors and probes (not shown) within the flexible bag, and for connecting one or more fluid lines to the flexible bag for fluids, gases, and the like, to be added or withdrawn from the flexible bag. Sensors/probes and controls for monitoring and controlling important process parameters include any one or more, and combinations of: temperature, pressure, pH, dissolved oxygen (DO), dissolved carbon dioxide (pCO₂), mixing rate, and gas flow rate, for example.

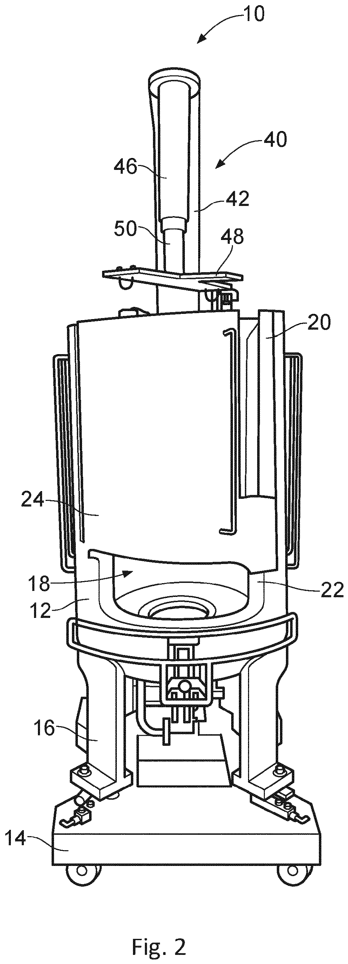

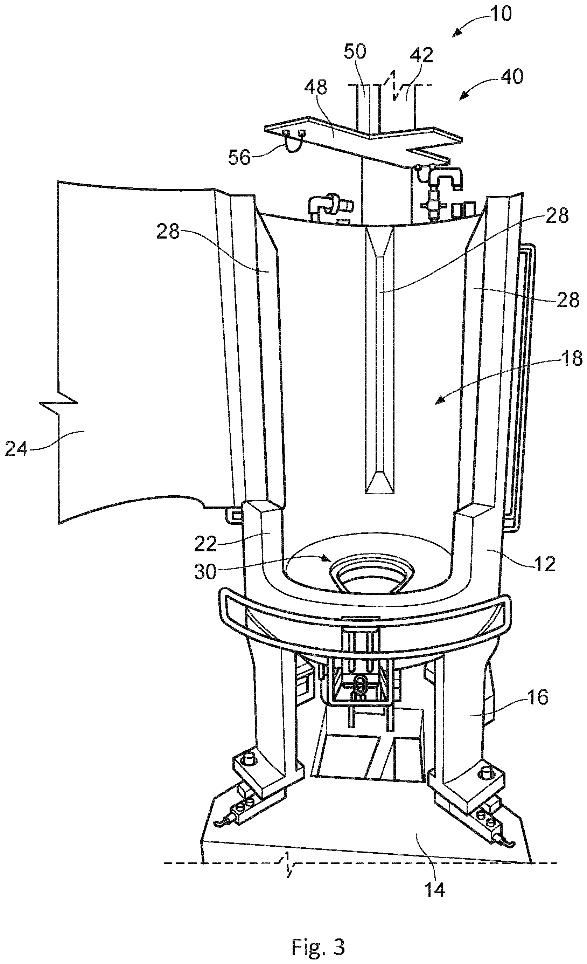

As best shown in FIGS. 2 and 3, in an embodiment, the vessel 12 includes an access door 24 hingedly or pivotally connected to a sidewall of the vessel 12 and moveable between a closed position (FIG. 2) and an open or access position (FIG. 3) permitting access to the interior space 18. The door 24 may include a handle 26 that facilitates movement of the door between the open and closed positions. In an embodiment, the door 24 may be configured and positioned such that when the door 24 is in the closed position, a lower edge of the door 24 forms an upper edge or boundary of the window 22, and/or a side edge of the door 24 forms an edge or boundary of the window 20. By having the edges of the door 24 define one or more boundaries of the windows 22, 24, when the door 22 is in the open position, a contiguous and unobstructed access opening in the sidewall of the vessel is formed by the opening 20, opening 22 and open door 24 (i.e., the opening in which the door is received). Accordingly, the area of the contiguous access opening formed in the sidewall of the vessel 12 when the door is in the open position is equivalent to the combined areas of the door 24, window 22 and window 24. This provides greater clearance and access to the interior space 18 than would otherwise be possible if the door and windows were separated by a portion of the sidewall of the vessel 12.

With further reference to FIG. 3, the interior sidewall of the vessel 12 may include one or more vertical baffles 28 that project into the interior space 18. The baffles 28 may be generally triangular in cross-section, although shapes and configurations known in the art may also be utilized without departing from the broader aspects of the invention. The baffles 28 are configured to contact and bias the flexible bag (when installed in the interior space 18) inwardly during a bioprocessing operation, for purposes known in the art. A bottom of the bioreactor vessel 12 includes a locating recess 30 for receiving an impeller base plate, as discussed in detail hereinafter.

As further shown in FIGS. 1-3, the bioreactor system 10 also includes a tubing and component management apparatus 40. The apparatus 40 includes a support member 42 mounted to the exterior sidewall of the vessel 12 and extending generally vertically above a top edge of the vessel 12. In an embodiment, the support member 42 may be mounted to the vessel by way of welding, bolts, screws, clamps or the like, although other means of attachment may also be utilized without departing from broader aspects of the invention. The apparatus 40 further includes a mast arm or boom 44 that extends generally horizontally from a distal end of the support member 42 and over the vessel 12, a guide sleeve 46 depending downwardly from a distal end of the boom 44, and a mounting plate 48 having a shaft 50 that is slidably received within the guide sleeve 46. In an embodiment, the apparatus 40 is configured such that the guide sleeve 46 and the shaft 50 of the mounting plate 50 are aligned with, i.e., coaxial with, a central axis or centerline of the vessel 12.

As best shown in FIG. 1, the apparatus 40 also includes a lifting mechanism 52 that is operable to selectively raise and lower the mounting plate 48 within the vessel 12, as in the directions indicated by arrow, A. For example, in an embodiment, the lifting mechanism may include a cable 54 connected to the shaft 50 of the mounting plate 48, and which extends through the guide sleeve 46, along (or within) the boom 44, and downward along (or within) the support member 42. A distal end of the cable is connected to an actuator which may be, for example, a hand crank, motor or other driving member. The actuator is operable to selectively retract the cable 54, thereby raising the mounting plate 48, or let out the cable 54, thereby lowering the mounting plate 48 into the vessel 12.

As illustrated in FIGS. 1-3, the mounting plate 48 may be generally T-shaped and includes a substantially planar upper surface for the mounting of various components used in bioprocessing operations, such as filters, filter heaters and other consumables. The mounting plate 48 may also include an array of slots or apertures configured to receive and retain various tubes configured for connection to the flexible bag received within the vessel 12. One or more hooks 56 (or other suitable coupling members) attached to the mounting plate 48 may be utilized to move and/or support the flexible bag.

In use, when installing a flexible bioprocessing bag prior to bioprocessing, the access door 24 in the sidewall of the vessel 12 may be opened, allowing for unobstructed access to the interior space 18 within the vessel 12. The lifting mechanism 52 may then be utilized to lower the mounting plate 48 into the vessel 12 to a height where it is easily accessible to an operator (for example, to about waist-height). At this point, the flexible bioprocessing bag (not shown) can be placed inside the vessel 12 and attached to the hooks 56 of the mounting plate 48. In addition, various tubes connected to the bag (or to be connected to the bag) can be organized and held out of the way of an operator by routing them through the slots/apertures in the mounting plate 48. Moreover, various functional components such as filter heaters, filters and other consumables can be attached to the mounting plate 48 such as by bolts. At this point, the actuator of the lifting mechanism 52 may be utilized to raise the mounting plate 48 to an operational position generally at the top of, or above, the vessel 12. The access door 24 can then be moved to a closed position and a bioprocessing operation commenced.

In an embodiment, the lifting mechanism 52 may interface with the control unit of the bioreactor system 10, such that upon selecting a 'start' or 'inflate' routine, the lifting mechanism 52 will automatically raise the mounting plate 52 to an operational position at the top of/above the vessel 12. The position of the mounting plate 48 can also serve as a position stop, limiting the extent to which the bag may be inflated. Similarly, at the end of a bioprocessing operation, selecting a 'deflate' or 'end' routine may automatically control the lifting mechanism 52 to lower the mounting plate 48. It is envisioned that, in some embodiments, lowering the mounting plate 48 may assist with deflation of the flexible bioprocessing bag, which has heretofore been a fairly time-consuming process. For example, lowering of the mounting plate 48 onto the bag may exert a downward force on the bag, assisting with deflation.

The bioreactor system 10 of the invention therefore provides an ergonomic means of installing the flexible bioprocessing bag, filters, filter heaters, and other consumables, and for organizing the various tubes connected to the bag. In contrast to existing systems, installation of such components can be carried out at waist-height from the side of the bioreactor vessel 12, obviating the need for multiple operators and stepladders.

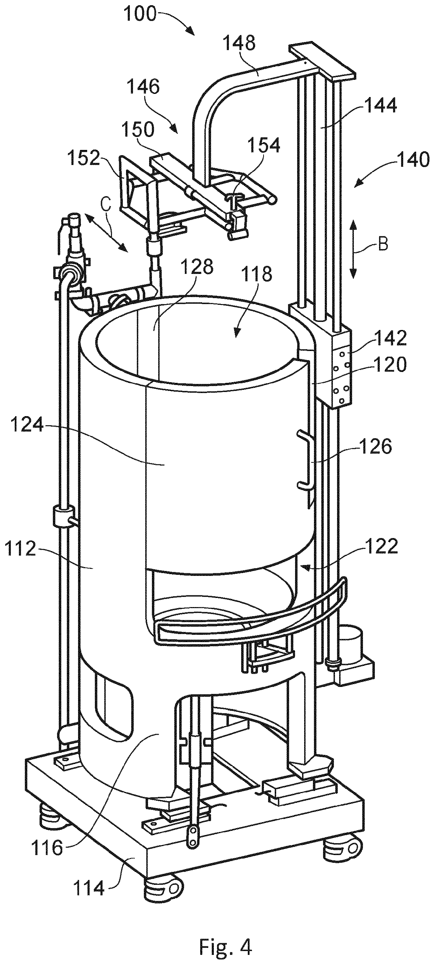

Turning now to FIGS. 4-9, a bioreactor system 100 is illustrated. The bioreactor system 100 is generally similar in configuration to the bioreactor system 10 of FIGS. 1-3 and includes a generally rigid bioreactor vessel or support structure 112 mounted atop a base 114 having a plurality of legs 116. The vessel 112, like vessel 12 is capable of supporting a single-use, flexible bioreactor bag in an interior space 118 thereof. The vessel 112 may likewise include one or more sight windows 120 and window 122 positioned at a lower area of the vessel 112, the purposes of which have been hereinbefore described.

Similar to the vessel 12 of FIGS. 1-3, the vessel 112 may also include an access door 124 hingedly or pivotally connected to a sidewall of the vessel 112 and moveable between a closed position and an open or access position permitting access to the interior space 118. The door 124 may likewise include a handle 126 that facilitates movement of the door between the open and closed positions. The door 124, as discussed above, may be configured so as to define a portion of a boundary of one or more of the windows 120, 122, maximizing the size of the opening in the sidewall of the vessel 112 when the door 124 is in the open position, as disclosed above.

With further reference to FIG. 4, the interior sidewall of the vessel 112 may include one or more vertical baffles 128 that project into the interior space 118, as discussed above.

As illustrated in FIG. 4, the bioreactor system 100, like bioreactor system 10, includes a tubing and component management apparatus 140 that facilitates installation and organization of tubing and consumable components of the bioreactor system 100. The apparatus 140 includes a linear actuator 142 mounted to an exterior sidewall of the vessel 112. The linear actuator 142 includes a shaft 144 that is selectively extendable and retractable in the vertical direction, denoted by arrow, B, as discussed in detail below. In an embodiment, the linear actuator 142 may take any form capable of moving the shaft 144 vertically such as, for example, a lead screw, a pneumatic actuator or a hydraulic actuator. In an embodiment, the actuator 142 may take the form of a cable and hand crank or motor-driven drive system, similar to that described above in connection with FIGS. 1-3. Other linear motion devices known in the art may also be utilized without departing from the broader aspects of the invention.

The apparatus 140 further includes a support frame 146 connected to the shaft 144 and moveable vertically therewith under control of the linear actuator 142. As illustrated in FIG. 4, the support frame 146 is suspended over the top opening of the bioreactor vessel 112 via a generally L-shaped support structure 148. The support frame 146 includes a guide rail 150, the purposes of which will be hereinafter described. The apparatus 140 also includes a mounting frame 152 that is slidably connected to the support frame 146 and, particularly, the guide rail 150 thereof. The mounting frame 152 is moveable in a horizontal direction away from, and towards, a central axis of the bioreactor vessel, as indicated by arrow, C. In an embodiment, a locking pin 154 is utilized to selectively restrain (or allow) horizontal movement of the mounting frame 152.

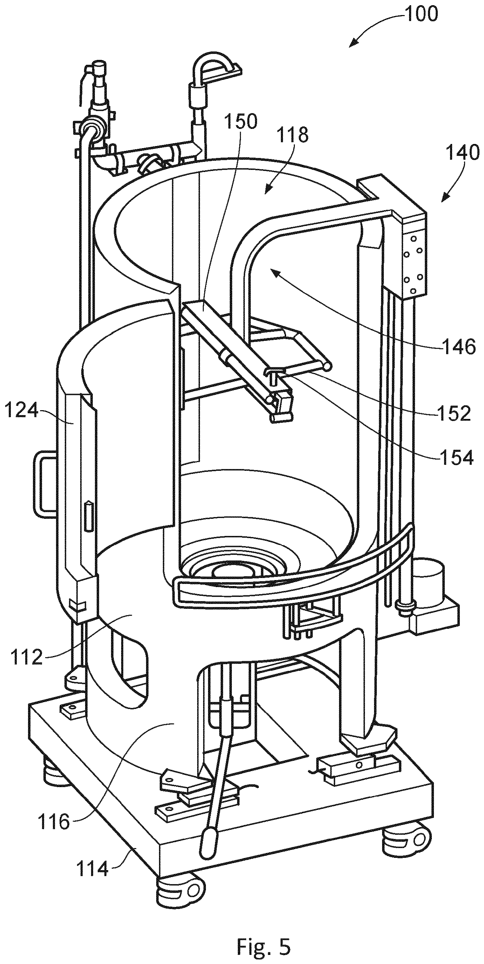

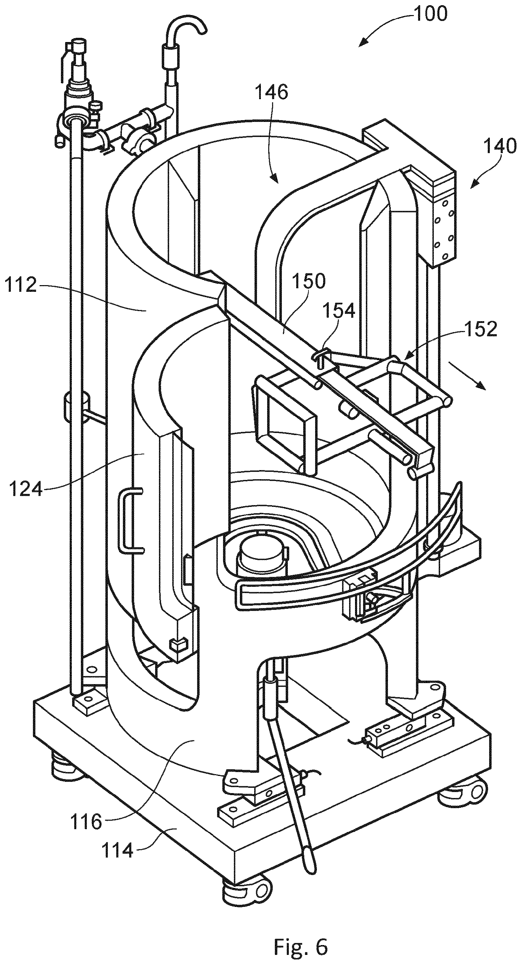

In use, when installing a flexible bioprocessing bag prior to bioprocessing, the tubing and component management apparatus 140 starts in an initial position where the linear actuator 142 is extended such that the mounting frame 152 is positioned above the top opening of the bioreactor vessel 112, as shown in FIG. 4. The linear actuator 142 is then utilized to lower the mounting frame 152 into the interior space 118, as illustrated in FIG. 5. The L-shaped support arm 148 of the support frame 146 is configured so that when in a lowered position, the mounting frame 152 is posited about waist-height off of the floor and ergonomically accessible to an operator. As further shown in FIG. 5, the door 124 may then be opened, providing easy access to the interior space 118. With reference to FIG. 6, the locking pin 154 can then be withdrawn, enabling the mounting frame 152 to move horizontally along the guide rail 150, to an extended position where the mounting frame 152 extends through the door opening and out of the interior space 118 of the vessel 112. The locking pin 154 can be utilized to lock the mounting frame 152 in its extended position, as also shown in FIG. 6.

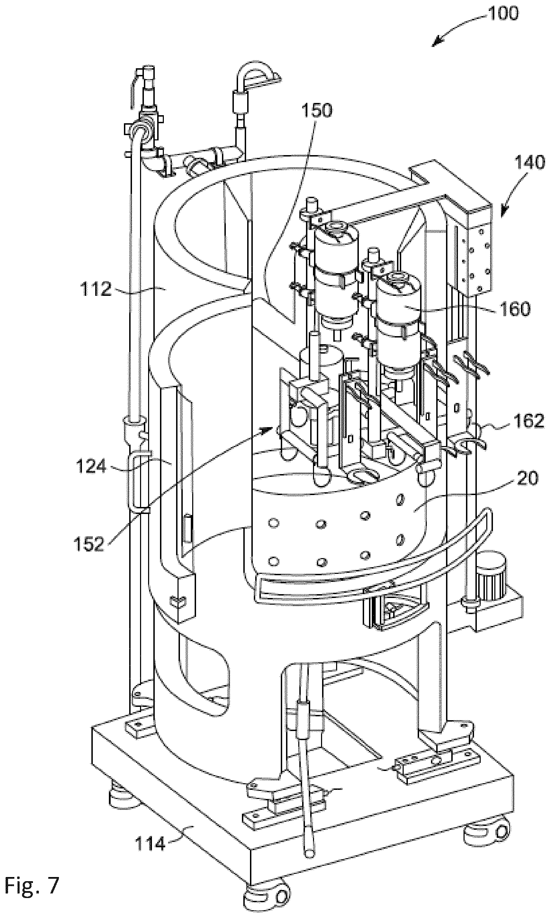

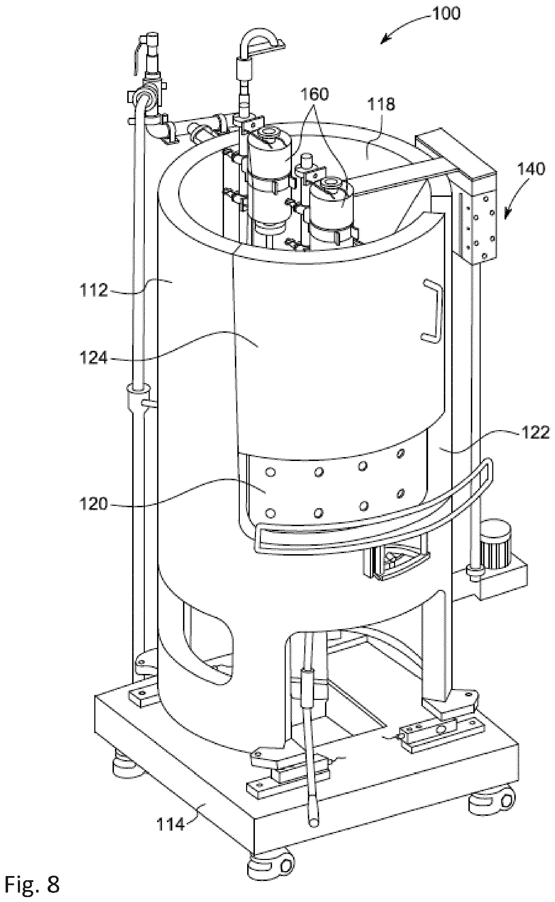

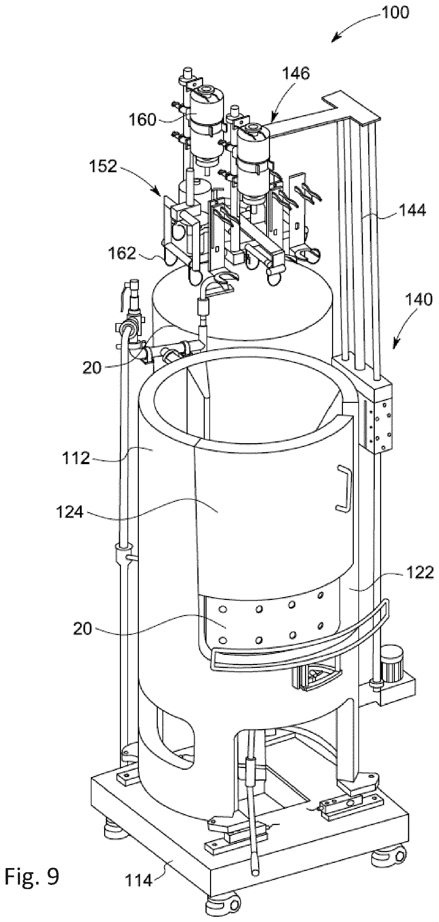

In this position, the mounting frame 152 is easily accessible for the mounting of consumable components including, for example, filters 160, filter heaters and the like to the mounting frame 152, as well as for the routing and management of tubing, as illustrated in FIG 7. As also shown therein, the flexible bioprocessing bag 20 can easily be installed in the interior space 118 and supported by the mounting frame 152 through one or more hooks 162 coupled to the mounting frame 152 (similar to those described above in connection with FIGS. 1-3). Once all of the tubing has been organized, the bag 20 installed, and various other components mounted securely to the mounting frame 152, the locking pin 154 is withdrawn and the mounting frame 152 is pushed back into position within the interior space 118 and the locking pin is inserted to retain the mounting frame 152 in such position. The door 124 is then closed, as shown in FIG. 8. With reference to FIG. 9, the linear actuator 142 is then utilized to move the mounting frame 152 and the attached components to an operational position at a required height above the bioreactor vessel 112 (i.e., generally outside of the interior space 118).

Similar to the embodiments disclosed above, in an embodiment, the apparatus 140 may be controlled by the control unit (not shown) of the bioreactor system 100 so that the mounting plate 152 can be automatically moved to an installation position (where the mounting plate 152 is extended through the door opening), an operational position (above the bioreactor vessel 112), or a deflating position (e.g., moving downwardly continuously or intermittently as the bag 20 is deflated) in dependence upon a selected mode of operation of the bioreactor vessel 100.

In addition to obviating the need for ladders and multiple operators to install the bioprocessing bag and other consumables, the bioreactor system 100 obviates the need of an operator to reach or lean into the interior space within the bioreactor vessel to install such components. In particular, the tubing and component management apparatus 140 is able to move vertically to a position where it can be easily accessed via a door in the sidewall of the vessel, without ladders, and the sliding mounting frame can be extended from the bioreactor vessel in the horizontal direction to provide an even greater ease of installation for such consumable components. This provides for easier and quicker installation, as well repeatability in the manner in which the bioprocessing bag is installed.

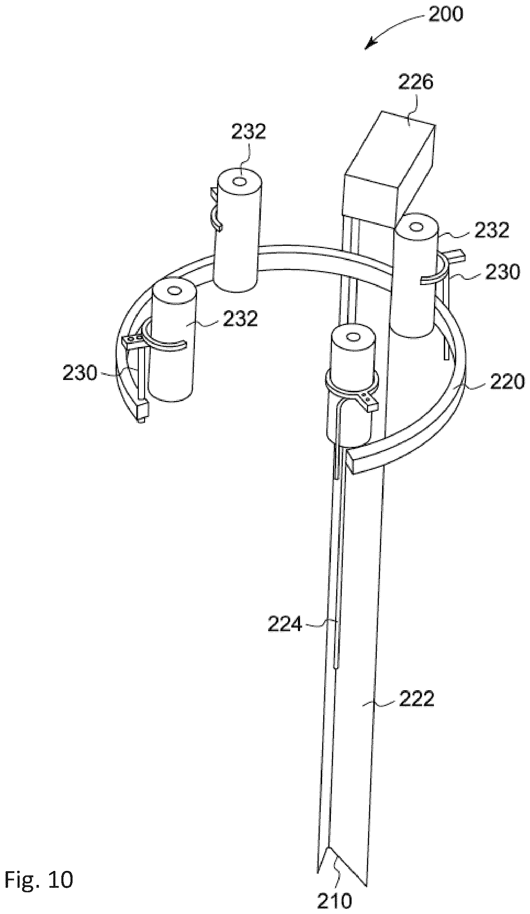

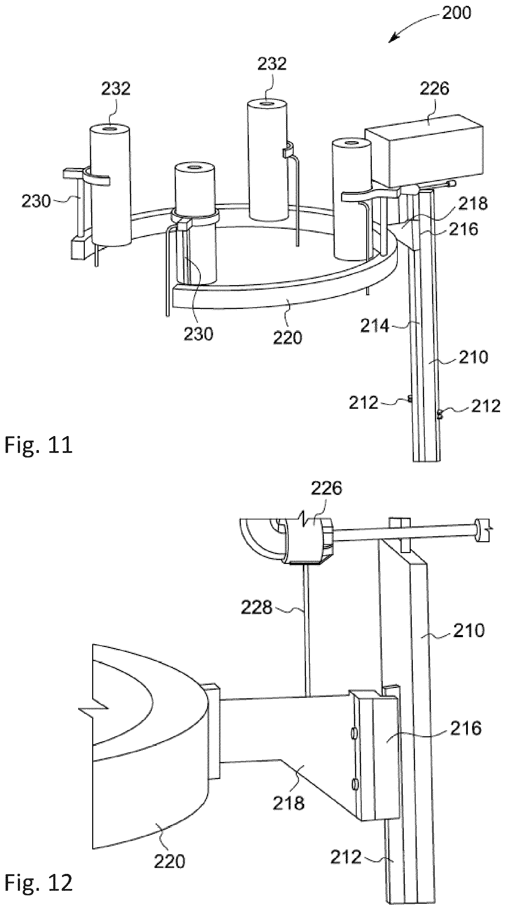

In an embodiment, the tubing and component management apparatus, rather than being mounted to the outside of the vessel as described above, may be integrated with one of the internal baffles (e.g., baffle 28 of vessel 10). FIGS. 10-13 illustrate one such implementation of a tubing and component management apparatus 200. The apparatus 200 includes a base plate 210 configured for mounting to the interior sidewall of a bioreactor vessel in place of one of the baffles. For example, in an embodiment, as shown in FIG. 11, the base plate 210 may include a plurality of flanges 212 having slots configured to receive threaded studs that protruding from the interior sidewall of a bioreactor vessel, for mounting of the base plate to the vessel using nuts that are received on the threaded studs.

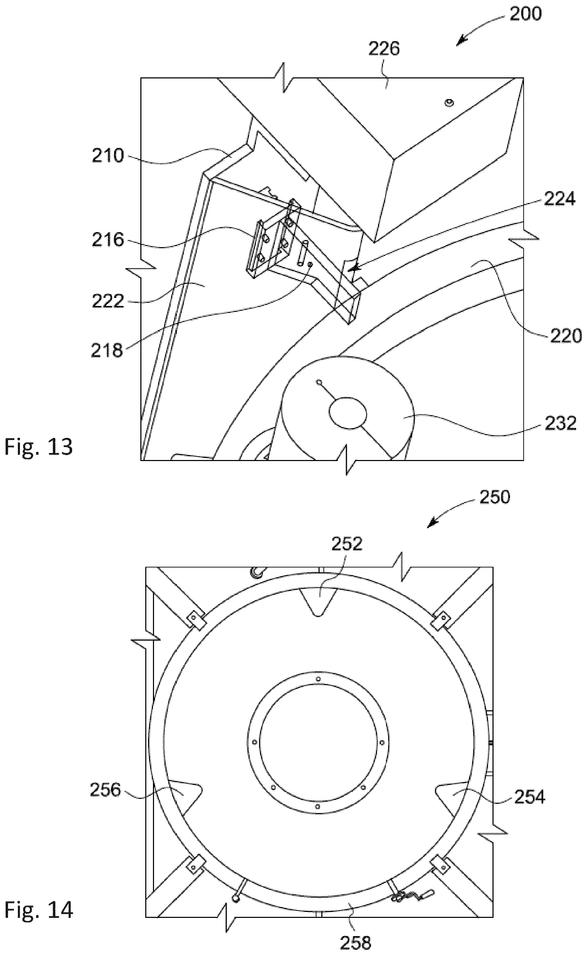

The apparatus 200 further includes a linear motion rail 214 coupled to the base plate 212, such as via bolts or other fasteners, a linear motion block 216 slidably coupled to the rail 214 for linear, vertical movement therealong, a carriage plate 218 coupled to the linear motion block 216, and a mounting frame 220 coupled to the carriage plate 218. As shown in FIGS. 10 and 13, a baffle cover 222 encloses the base plate 210, the rail 214 and the sliding block 216 and defines a hollow interior space between the cover 222 and the base plate 212. The baffle cover 222 is shaped so as to provide a substantially equivalent function and performance as typical baffles, and may be, for example, generally triangular in cross section. As best shown in FIGS. 10 and 13, the baffle cover 222 includes a slot 224 in an apex thereof, through which the carriage plate 218 extends. The hollow interior and the slot 224 allow for vertical movement of the sliding block 216, carriage plate 218 and mounting frame 220 along the guide rail 214, as disclosed hereinafter.

As illustrated in FIGS. 10-12, the apparatus 200 also includes a cable hoist 226 or other driving mechanism having a cable 228 connected to the carriage plate 218. The cable hoist 226 may be hand driven or motor driven, and is actuatable to let out the cable 228 or retract the cable 228 to selectively raise or lower the sliding block 216, and thus the carriage plate 218 and mounting frame 220, along the rail 214, to adjust a vertical position of the mounting frame 220.

As shown in FIGS. 10-13, the mounting frame 220 may be generally annular or semi-annular in shape, although other shapes and configurations are also envisioned. The mounting frame 220 may include a plurality of filter holder devices 230 for receiving and retaining filters 232, as well as bag hooks, tubing holders, and similar mounting mechanisms for mounting of an array of consumable components to the mounting frame 220.

FIG. 14 is a top plan view illustrating an exemplary bioreactor vessel 250 with which the tubing and component management apparatus 200 may be utilized. As illustrated therein, the vessel includes a plurality of internal baffles 252, 254, 256. In an embodiment, baffle 25, opposite access door 258, may be replaced by the tubing and component management apparatus 200. The tubing and component management apparatus 200, as disclosed above, may be easily secured to the vessel sidewall, as well as easily removed during transportation as well as in the field, for service. As illustrated in FIG. 10, the top of the mounting frame 220 is free from any cables or structures, which makes it easier to install the filters.

Similar to the embodiments disclosed above, the apparatus 200 may be controlled by the control unit (not shown) of the bioreactor system so that the mounting frame can be automatically lowered to an installation position before commencement of a bioprocessing operation, and raised to an operational position upon commencement of such operation.

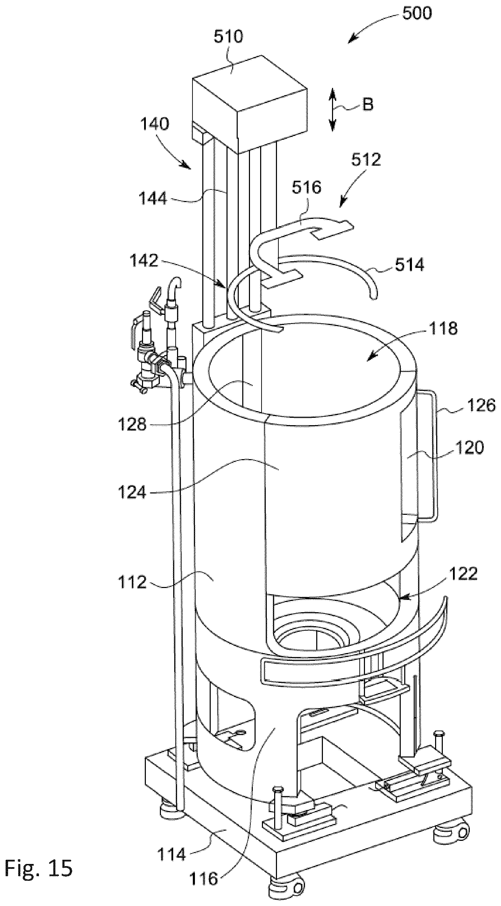

FIGS. 15-19 depict another a bioreactor system 500 that is generally similar in configuration to the bioreactor system 100 of FIGS. 4-9, where like reference numerals designate like parts. As illustrated therein, the tubing and component management apparatus 140, however, includes a slightly different mounting frame for the mounting of various consumable components, tubes, and the like. In particular, the apparatus 140, at the top of the shaft 144 of the linear actuator 140 includes a connector box 510, and a floating frame 512 connected to the connector box 510. The floating frame 512 includes a lower frame member 514 that is semi-annular in shape and is configured to support at least one consumable component such as, for example, the flexible bioprocessing bag 20. The floating frame 512 additional includes an upper frame member 516 that is likewise semi-annular in shape and is configured to support at least one consumable component such as, for example, filters 160 and a pinch valve assembly 518. The floating frame 512, and the frame members 514, 516 thereof, are mounted so as to be moveable vertically into and out of the interior space 118 within the bioreactor vessel 112 in the manner described above.

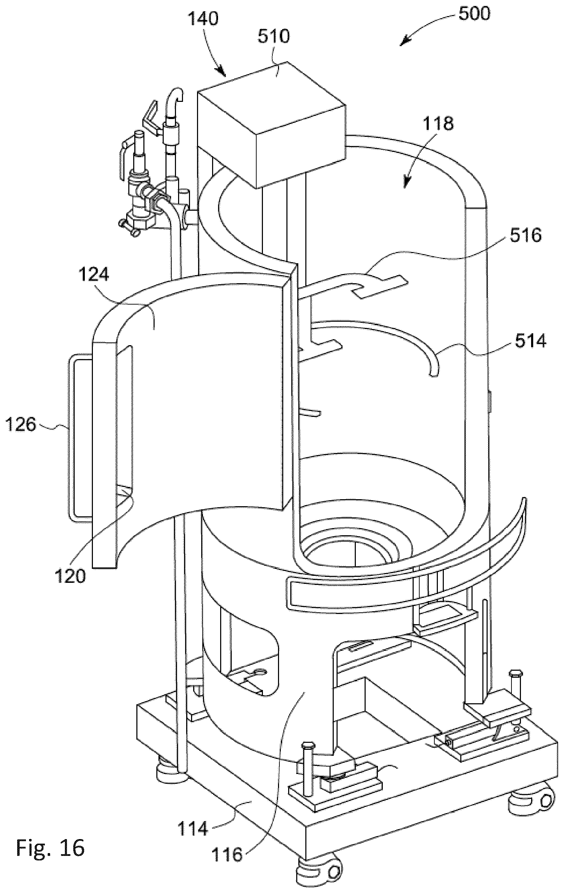

In particular, in use, when installing a flexible bioprocessing bag 20 prior to bioprocessing, the tubing and component management apparatus 140 starts in an initial position where the linear actuator 142 is extended such that the floating frame 512 and the frame members 514, 516 thereof are positioned above the top opening of the bioreactor vessel 112, as shown in FIG. 15. The linear actuator 142 is then utilized to lower the floating frame 512 into the interior space 118, as illustrated in FIG. 16. The door 124 can then be opened (although it envisioned that it can be opened prior to lowering the floating frame 512).

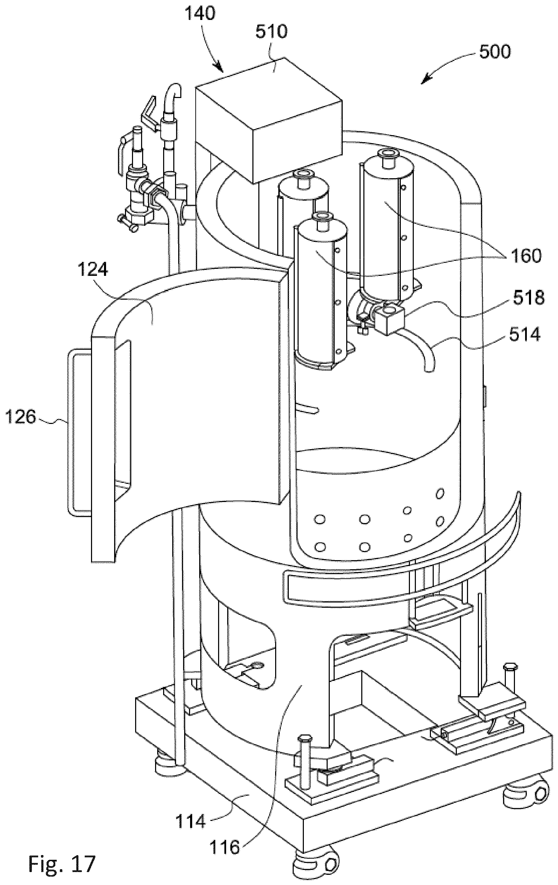

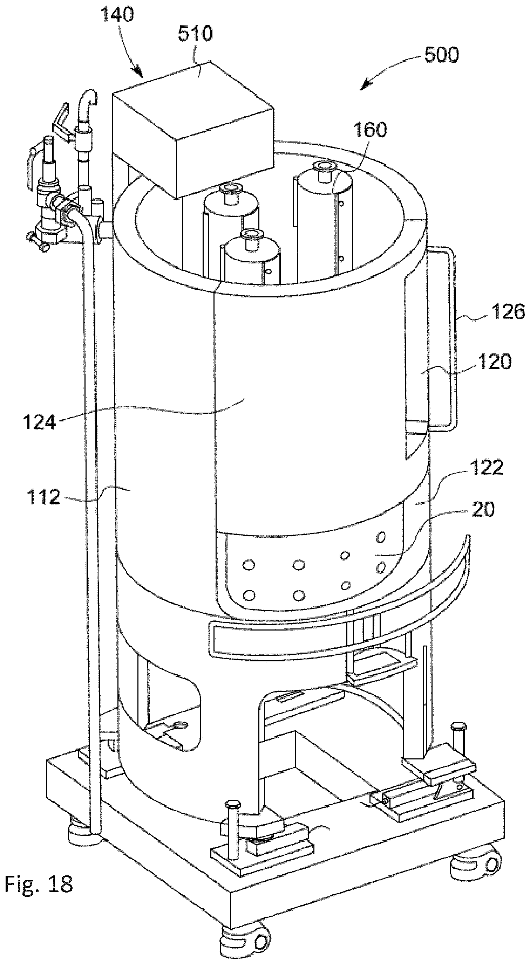

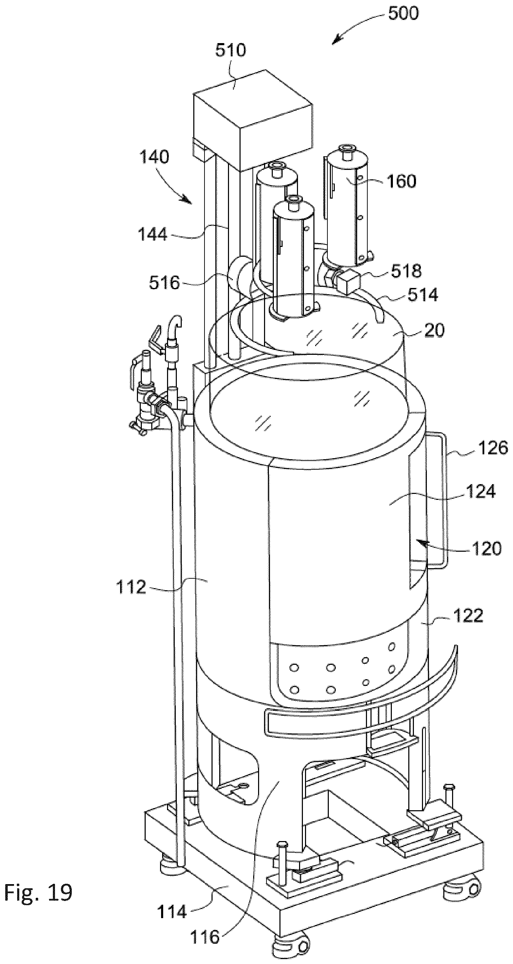

In this position, the floating frame 512 is easily accessible through the door opening for the mounting of consumable components including, for example, filters 160, filter heaters, the flexible bag 20, and a pinch valve assembly 518 for tubing, and the like, to the upper and lower frame members 514, 516, as well as for the routing and management of tubing, as illustrated in FIG 17. As also shown therein, the flexible bioprocessing bag 20 can easily be installed in the interior space 118 and supported by the lower frame member 514. Once all of the tubing has been organized, the bag 20 installed, and various other components mounted securely to the floating frame 512, the door 124 is then closed, as shown in FIG. 18. With reference to FIG. 19, the linear actuator 142 is then utilized to move the floating frame 512 and the attached components to an operational position at a required height above the bioreactor vessel 112 (i.e., generally outside of the interior space 118).

Similar to the embodiments disclosed above, in an embodiment, the apparatus 140 may be controlled by the control unit (not shown) of the bioreactor system 500 so that the floating frame 512 can be automatically moved to an installation position (where the floating frame is received within the interior space 118 at about waist-height of an operator), an operational position (above the bioreactor vessel 112), or a deflating position (e.g., moving downwardly continuously or intermittently as the bag 20 is deflated) in dependence upon a selected mode of operation of the bioreactor vessel/bioprocessing system 500.

The embodiments of the tubing and component management apparatus described herein provide for an ergonomic means of installing the flexible bioprocessing bag, filters, filter heaters, and other consumables, and for organizing the various tubes connected to the bag. In contrast to existing systems, installation of such components can be carried out at waist-height from the side of the bioreactor vessel 12, obviating the need for multiple operators and stepladders.

As indicated above, in addition to present difficulties installing the flexible bioprocessing bag and other components at the top of the bioreactor vessel, properly seating the impeller base plate of the flexible bioprocessing bag on the bottom of the bioreactor vessel during installation of the flexible bag may also present challenges. Accordingly, embodiments of the invention, in addition to providing for tubing and component management for the top of the flexible bag (namely, for tubing and components mounted above the flexible bag at the top of the bioreactor vessel), also provide for management of components at the bottom of the flexible bag. In particular, embodiments of the invention are directed to locating, locking and retaining mechanisms for locking the impeller base plate within the recess (e.g., the impeller base plate recess 30 of FIG. 3), in the bottom of the bioreactor vessel, and an indicating mechanism for indicating that the base plate is properly positioned within the recess.

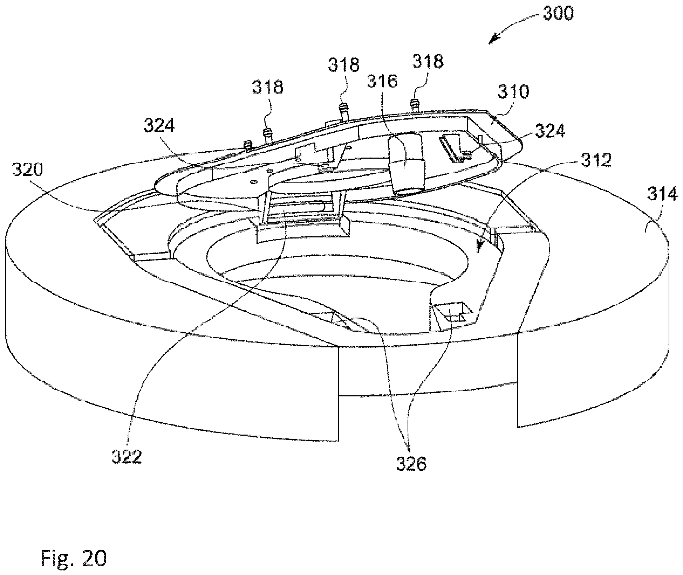

With reference to FIG. 20, a component management system 300 in the form of a locking mechanism for an impeller base plate 310 for a bioprocessing system is shown. The system 300 includes a base plate 310 which may be attached to the bottom of a flexible, single-use bioprocessing bag (not shown) for use in stirred-tank bioreactor systems like that shown in FIGS. 1-19. The base plate 310 may be mounted within an opening of the bottom of the flexible bioprocessing bag such as by welding, although other means of attachment may also be utilized without departing from the broader aspects of the invention. As is known, the base plate is configured to be received in a corresponding base plate recess 312 in the bottom 314 of a bioreactor vessel. As is known, the base plate 310 serves as an interface between an impeller (not shown) mounted to the base plate 310 interior to the flexible bag, and a rotating magnetic drive head (not shown) outside of the bag beneath the base plate 310. The base plate 310 may also include a harvest port 316 for the connection of drain tubing to drain the flexible bag, and a plurality of ports 318 for the mounting of a sparger to the base plate 310 interior to the flexible bag.

As shown in FIG. 20, in the inventive embodiment, the base plate 310 also includes a locating mechanism in the form of a slot 320 that extends from the underside of the base plate 310. The slot 320 is configured to receive a corresponding tongue 322 that projects into a rearward portion of the recess 312 from the vessel bottom 314. In an embodiment, it is contemplated that components of the locating mechanism may be reversed, such that the base plate may have a tongue projection that is received in a corresponding slot or groove in the vessel bottom 312.

With further reference to FIG. 20, the base plate 310 also includes a latching mechanism on the underside thereof opposite the slot 320. In an embodiment, the latching mechanism includes a pair of downwardly depending latches 324 that are configured to be received by a corresponding locking/latching mechanism in the vessel bottom 314, namely, in latch openings 326 in the vessel bottom 314. In an embodiment, the latches 324 are generally L-shaped and are resilient such that when the base plate 310 is rotated downwardly about the tongue 322, the base plate 310 is snapped into seated position within the recess 312 and the latches 324 engage the vessel bottom 314 to lock the base plate 310 in position. The latches 324 may be accessed beneath the vessel and pinched inwardly to release the base plate 310 from the recess 312. While FIG. 20 illustrates two latches 326, more than two latches, or a single latch, may be utilized without departing from the broader aspects of the invention.

In use, the flexible bag is inserted into the bioreactor vessel and the base plate 310 is angled as illustrated in FIG. 20 such that the tongue 322 is received in the slot 320 of the base plate 310. The front of the base plate 310 is then urged downwardly until the latches 326 are received in the recesses 326 and snap into place to retain the base plate 310 in the recess. This ensures the base plate is constrained in all three axes.

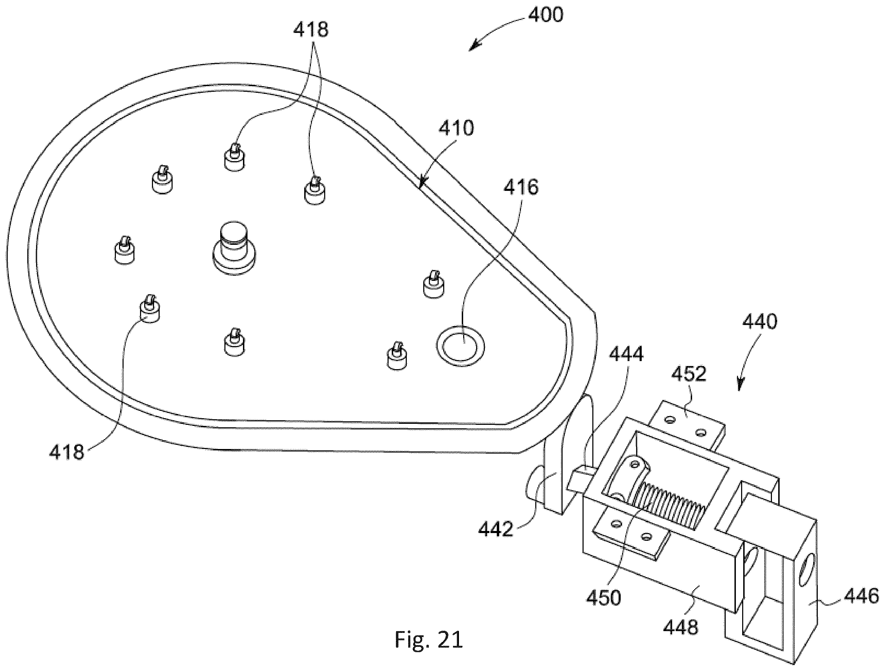

Turning now to FIGS. 21-23 another component management system 400 in the form of a locking/latching mechanism for an impeller base plate, according to another embodiment of the invention is shown. The system 400 includes a base plate 410 which may be attached to the bottom of a flexible, single-use bioprocessing bag as described above, and which is configured to be received in a corresponding base plate recess in the bottom of a bioreactor vessel. As is known, the base plate 410 may include a harvest port 416 for the connection of drain tubing to drain the flexible bag, and a plurality of ports 418 for the mounting of a sparger to the base plate 410 interior to the flexible bag.

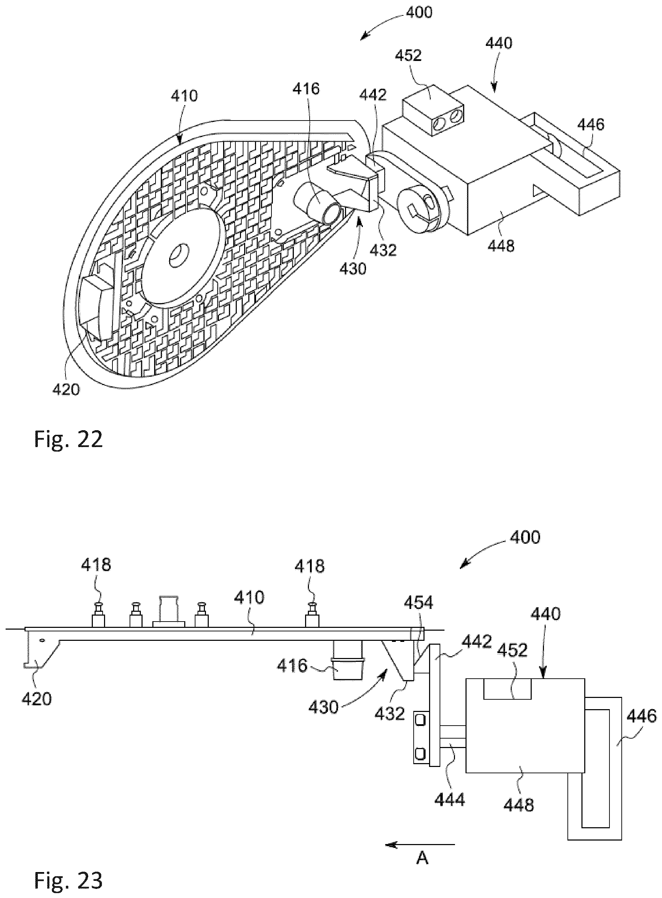

Similar to the base plate 310 of FIG. 20, the base plate 410 includes a rear slot 420 that extends from the underside of the base plate 410, and which is configured to receive a corresponding tongue (not shown) that projects into a rearward portion of the recess in the bottom of the bioreactor vessel. As best shown in FIGS. 22 and 23, the base plate 410 also includes a forward catch 430 that extends downwardly from an underside of the base plate 410. In an embodiment, the catch 430 is generally U-shaped or L-shaped having a catch member 432 that lies in a plane generally parallel to a body of the base plate 410.

As best shown in FIGS. 21-23, the system 400 also includes a latch mechanism 440 having a latch member 442 that is configured to engage the catch member 432 of the catch 430. As illustrated therein, the latch member 442 is attached to the distal end of an elongate shaft 444, opposite a handle 446. The shaft 444 extends through a housing 448 and is connected to a spring, e.g., coil spring 450, within the housing 448. The spring 450 biases the latch member 442 away from the housing 448 and towards the catch member 432 on the underside of the base plate 410, as discussed hereinafter. The housing 448 is configured for mounting to the exterior bottom of the bioreactor vessel adjacent to the base plate recess. For example, in an embodiment, the housing 448 may include mounting flanges 452 for attaching the housing 448 to the bottom of the bioreactor vessel using bolts or screws.

As best shown in FIG. 23, in an embodiment, the latch member 442 may be offset from a longitudinal axis defined by the shaft 444. As also shown therein, in an embodiment, the latch member 442 may have a sloped or angled contact surface 454. This surface functions to translate a downward force exerted on the latch member 442 by the catch member 432 into a horizontal force that urges the shaft 44 rearwardly against the spring bias of the coil spring 450, as discussed hereinafter.

In use, the flexible bag is inserted into the bioreactor vessel and the base plate 410 is angled such that the tongue that projects into the recess in the bottom of the bioreactor vessel is received in the slot 420 of the base plate 410. The front of the base plate 410 is then urged downwardly until the bottom of the catch member 432 contacts the angled surface 454 of the latch member 442 of the latch mechanism 440. Continued downward urging of the base plate 410 causes the catch member 432 to exert a force on the angled surface 454 of the latch member 442, causing the latch member 442 and the shaft 444 to move rearwardly against the spring bias of the coil spring 450. As the catch member 432 passes the lower edge of the angled surface 454, the spring bias of the coil spring 450 causes the shaft 444 and latch member 442 to translate forwardly, in the direction of arrow, A, in FIG. 23. In this position, the latch member 442 extends over the catch member 432, locking the base plate 410 in position within the recess. This ensures the base plate is constrained in all three axes. In an embodiment, the handle 446 may include a visual feature such as visible demarcation lines or features to indicate a locked and unlocked state of the base plate 410.

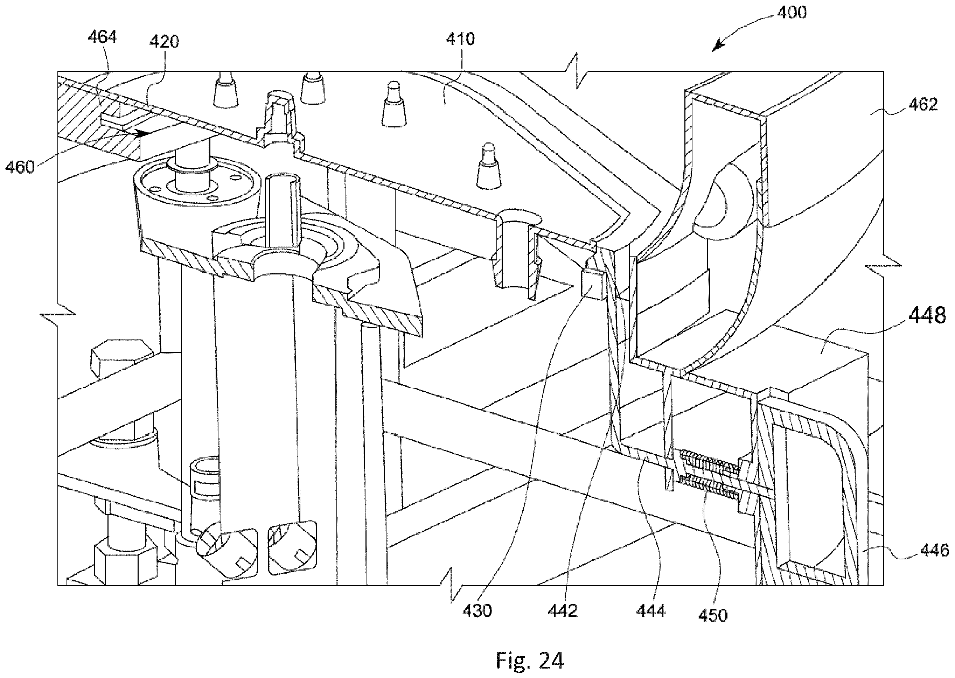

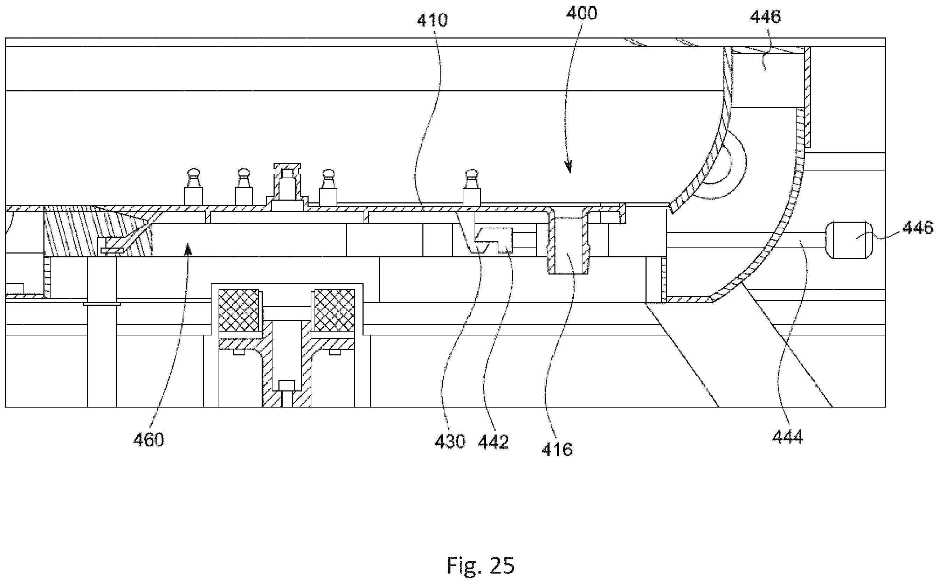

FIGS. 24 and 25 are cross sectional views of the base plate 410 in locked position within a recess 460 in the bottom of a bioreactor vessel 462. As shown, the tongue 464 in the bottom of the vessel 462 is received in the slot 420, and the latch member 442 engages the catch 430 to retain the base plate 410 in the recess 460.

During unloading of the flexible bag, an operator may simply pull on the handle 446 to move the shaft 444 and latch member 442 against the spring bias, to a position in which the latch member 442 does not engage the catch 430. In this position, the base plate 410 may be freely rotated out of the recess and removed.

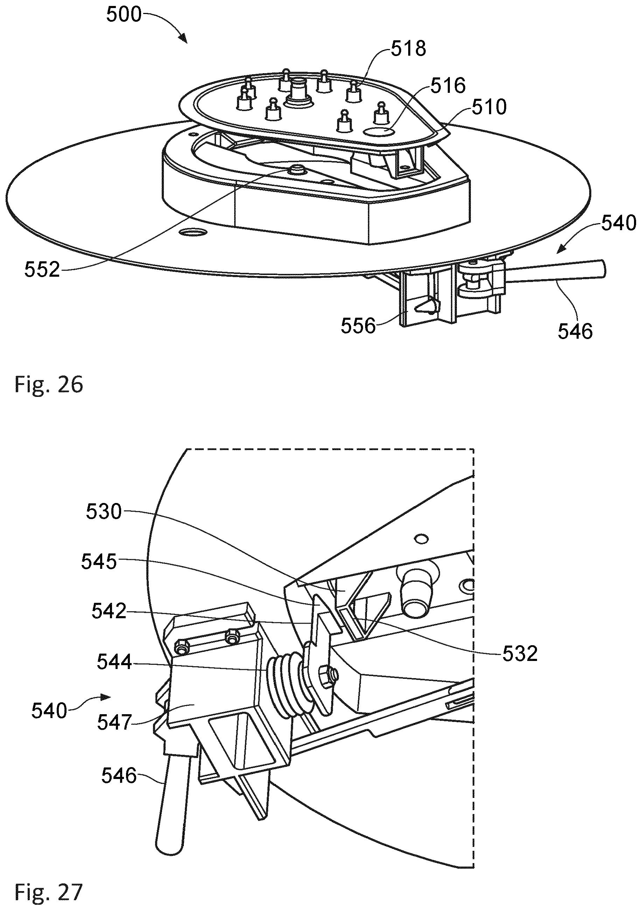

Turning now to FIGS. 26-29 another component management system 500 in the form of a locking/latching mechanism and indicator for an impeller base plate, according to another embodiment of the invention is shown. The system 500 includes a base plate 510 which may be attached to the bottom of a flexible, single-use bioprocessing bag as described above, and which is configured to be received in a corresponding base plate recess in the bottom of a bioreactor vessel. As is known, the base plate 510 may include a harvest port 516 for the connection of drain tubing to drain the flexible bag, and a plurality of ports 518 for the mounting of a sparger to the base plate 510 interior to the flexible bag.

Similar to the base plate 310 of FIG. 20 and base plate 410 of FIG. 21, the base plate 510 may include a rear slot (not shown) that extends from the underside of the base plate 510, and which is configured to receive a corresponding tongue (not shown) that projects into a rearward portion of the recess in the bottom of the bioreactor vessel. As best shown in FIGS. 27-29, the base plate 510 also includes a forward catch 530 that extends downwardly from an underside of the base plate 510. In an embodiment, the catch 530 is generally U-shaped or L-shaped having a catch member 532 that lies in a plane generally parallel to a body of the base plate 510.

As best shown in FIGS. 27-29, the system 500 also includes a latch mechanism 540 having a latch member 542 that is configured to engage the catch member 532 of the catch 530. As illustrated therein, the latch member 542 is attached to the distal end of a shaft 544, opposite a handle 546. The shaft 544 extends through a housing 547 and is connected to handle 546. The housing 547 is configured for mounting to the exterior bottom of the bioreactor vessel adjacent to the base plate recess, as shown in FIG. 27. For example, in an embodiment, the housing 547 may include mounting flanges for attaching the housing 547 to the bottom of the bioreactor vessel using bolts or screws.

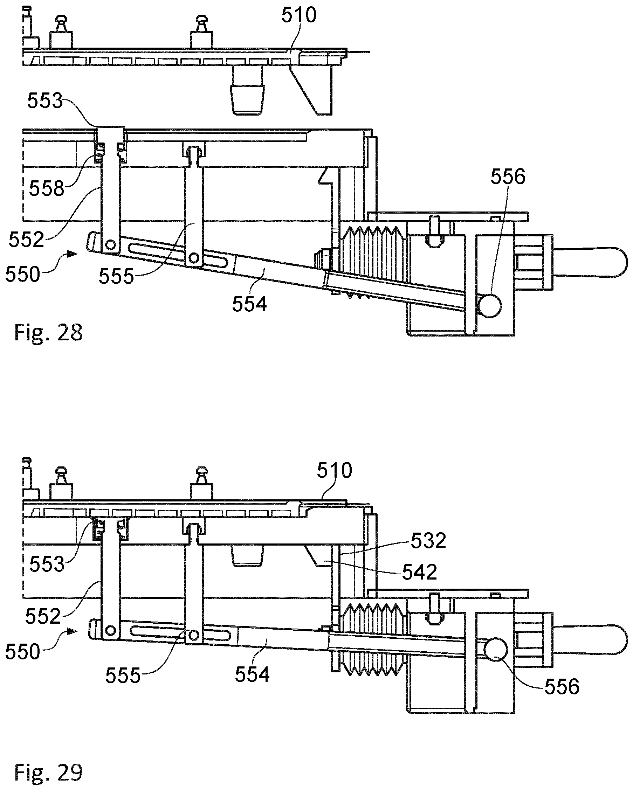

As best shown in FIG. 27, in an embodiment, the latch member 542 may be offset from a longitudinal axis defined by the shaft 544. As also shown therein, in an embodiment, the latch member 542 may have a sloped or angled contact surface 545. In order to lock the base plate 510 to the bottom of the bioreactor vessel, a user can rotate handle 546, which cause shaft 554 to extend, resulting in latch member 542 engaging with catch member 532, as best illustrated in FIGS. 28-29.

The mechanical locking system 500 for the impeller base plate as described above further includes an indicator mechanism 550. As best shown in FIGS. 26, 28, and 29, the indicator mechanism 550 includes a plunger 552 that extends through the bottom of the bioreactor vessel into the recess. The plunger 552 is attached to a rocker arm 554 that further extends into housing 547. Located at the end of rocker arm 554 is an indicator 556. As best shown in FIGS. 26, 28, and 29, the indicator is a portion of rocker arm 554 or a pointer (or other visual indicating means) attached to an end of rocker arm 554 (e.g., a cone shaped attachment) along with an indicator panel that is attached to, or a portion of, housing 547, as illustrated by FIG. 26. The indicator panel provides two visual indication regions (i.e., a top portion and a bottom portion), corresponding to a proper positioning and an improper positioning of the base plate 510. For example, the top portion may be colored green, while the bottom portion may be colored red.

In use, prior to attachment of the base plate 510 to the bottom of the bioreactor vessel, plunger 552 is in an extended position such that the top 553 of the plunger 552 protrudes into the recess in the bioreactor vessel. This is accomplished via a biasing mechanism 558 (e.g., a spring) located in a cutout in the bioreactor vessel. In this configuration, and when the base plate 510 is improperly positioned, rocker arm 554 slopes downwardly, such that the indicator 556 is located on the bottom portion of the indicator panel, as best illustrated by FIG. 28. When the base plate 510 is properly positioned the plunger 552 is pushed down into the recess, which causes rocker arm 554 to pivot about a pivoting arm 555, which raises the indicator 556 relative to the indicator panel, as best illustrated by FIG. 29. In this way, the indicator 556 moves up and down on the indicator panel, providing an indication of whether the base plate 510 is properly positioned.

According to alternative embodiments, the indicator mechanism 500 includes a sensor associated with, or configured to replace, the plunger. The sensor is configured to indicate when the base plate is properly positioned. By way of example, the sensor can be a proximity sensor that emits a signal (e.g., light, electromagnetic radiation) that is configured to indicate when the base plate is properly position. By way of a further example, the sensor can be a mechanical sensor (e.g., a mechanical switch) that is depressed/actuated upon proper placement of the base plate. An output signal from sensor is configured to provide an indication (e.g., visual, tactile, or auditor) when proper placement occurs.

According to further alternative embodiments, the rocker arm is configured to move from a position in which it prevents the locking/latching mechanism from engaging the base plate to a position in which the locking/latching mechanism is free to engage the base plate, corresponding to an improper and proper position of the base plate within the recess, respectively. By way of example, the rocker arm may include a portion that is configured to abut a portion of the locking/latching mechanism of any of the aforementioned embodiments, such that when the base plate is improperly positioned the rocker arm prevents the locking mechanism from properly engaging the base plate (e.g., prevents handle 546 fully swinging into position). Only when the base plate is properly position, and thereby the rocker arm moves, is the locking mechanism allowed to engage the base plate.

It is noted that the location of plunger 552 (or sensor) within the recess of the bottom of the bioreactor vessel, according to embodiments, is generally centrally located. According to a preferred embodiment, and as illustrated by FIG. 26, the plunger is located adjacent to a cutout in the bottom of the bioreactor vessel close to the center of recess. This is a preferred location, as it ensures that the plunger is only depressed when the base plate 510 is properly placed. If the plunger is peripherally placed there is a chance that it may be depressed even when the base plate 510 is improperly placed. Advantageously, indicator mechanism 550 provides a clear indication of whether the base plate is properly positioned within the recess. The indicator mechanism further addresses potential problems associated with the difficulties in knowing whether the base plate is properly positioned, even when the front or back of the base plate is latched within the recess.

The mechanical locking systems for the impeller base plate described herein provide a means for securely locking the impeller base plate in the recess of the bottom of the bioreactor vessel during installation of the flexible bag. In addition, the mechanisms hereinbefore described provide a tactile, visual or other indication that the base plate is in seated and locked position within the recess. As also described above, while securely locked to the vessel, the base plate can still be easily removed upon completion of a bioprocessing operation.

It is contemplated that the base plate locking system described herein in connection with FIGS. 20-29 may be incorporated into any stirred tank bioreactor vessel known in the art. Still further, it is contemplated that the base plate locking systems of the invention may be incorporated into any the bioreactor vessels illustrated in FIGS. 2-19, which include a tubing and component management system, as described herein. In particular, in an embodiment, a bioreactor vessel may include one of, or both of, a base plate locking mechanism for securing the base plate to the bottom of the bioreactor vessel, and a tubing and component management system for arranging and securing consumable components above the flexible bag. The combination of the base plate locking mechanism and the tubing and component management system provides for a bioreactor vessel that facilitates installation of consumable components and provides for an ease of use heretofore not seen in the art.

In an embodiment, a bioreactor vessel is provided. The bioreactor vessel includes a bottom, a peripheral sidewall, the bottom and the peripheral sidewall defining an interior space for receiving a flexible bioprocessing bag, a recess in the bottom for receiving a base plate of the flexible bioprocessing bag, a locking mechanism configured to retain the base plate in the recess, and an indicator mechanism configured to indicate when the base plate is properly positioned in the recess. In an embodiment, the locking mechanism includes a latch, wherein the latch is moveable between an engagement position where the latch engages the base plate when the base plate is positioned in the recess in the bottom of the bioreactor vessel to retain the base plate in the recess, and a clearance position where the base plate can be withdrawn from the recess. In an embodiment, the latch is spring-biased toward the engagement position. In an embodiment, the locking mechanism includes a handle that is operable to move the latch from the engagement position to the clearance position. In an embodiment, the latch includes an angled upper surface configured to translate a downward force from the base plate into a lateral force for moving the latch to the clearance position against the spring-bias during installation of the base plate in the recess. In an embodiment, the indicator mechanism includes a plunger, a rocker arm, and an indicator. In an embodiment, the plunger initially protrudes into the recess. In an embodiment, when the base plate is properly placed in the recess, the indictor moves in a direction opposite to movement of the plunger via the rocker arm, which provides the indication of proper placement. In an embodiment, the bioreactor vessel includes a tongue extending into the recess opposite the locking mechanism, the tongue being configured to engage a slot in a rear area of the base plate. In an embodiment, the locking mechanism includes at least one aperture adjacent to the recess and configured to receive at least one corresponding latch of the base plate. In an embodiment, the at least one aperture is a pair of apertures. In another embodiment, a bioprocessing apparatus is provided. The apparatus includes a flexible bioprocessing bag, and a base plate positioned at a bottom of the flexible bioprocessing bag and being shaped so as to be received in a corresponding recess in a bottom of a bioreactor vessel. The base plate includes a locating mechanism adjacent to a rear edge of the base plate, for cooperating with a corresponding locating feature on the bottom of the bioreactor vessel adjacent to the recess to locate the base plate in the recess, and a locking mechanism extending downwardly from an underside of the base plate opposite the locating mechanism, for cooperating with a corresponding locking device of the bioreactor vessel for retaining the base plate in the recess. In an embodiment, the locating mechanism is one of a slot and a tongue, and the corresponding locating feature is the other of a slot and a tongue. In an embodiment, the locking mechanism is a catch lying in a plane generally parallel to, and spaced from, a body of the base plate, and the locking device includes a latch configured to engage the catch. In an embodiment, the locking mechanism is at least one latch member, and the locking device includes a recess configured to receive the at least one latch member. In an embodiment, the at least one latch member is generally L-shaped. In an embodiment, the at least one latch member is resilient.

In yet another embodiment, a bioprocessing system is provided. The bioprocessing system includes a bioreactor vessel having a bottom and a peripheral sidewall defining an interior space, a recess in the bottom, a locking mechanism adjacent to the recess, an indicator mechanism, and a flexible bioprocessing bag positionable within the interior space, the flexible bioprocessing bag including a base plate at a bottom of the flexible bioprocessing bag. The base plate is configured to be received in the recess in the bottom of the bioreactor vessel. The locking mechanism is configured to engage the base plate to retain the base plate in the recess. In an embodiment, the locking mechanism includes a latch, and the base plate includes a catch. The latch is moveable between an engagement position where the latch engages the catch when the base plate is positioned in the recess to retain the base plate in the recess, and a clearance position where the base plate can be withdrawn from the recess. In an embodiment, the latch is spring-biased toward the engagement position. In an embodiment, the locking mechanism includes a handle that is operable to move the latch from the engagement position to the clearance position. In an embodiment, the latch includes an angled upper surface configured to translate a downward force from the catch of the base plate into a lateral force for moving the latch to the clearance position against the spring-bias during installation of the base plate in the recess. In an embodiment, the system may also include a tongue extending into the recess opposite the locking mechanism, the tongue being configured to engage a slot in a rear area of the base plate. In an embodiment, the locking mechanism includes at least one aperture adjacent to the recess and configured to receive at least one latch depending downwardly from the base plate opposite the slot. In an embodiment, the indicator mechanism includes a plunger, a rocker arm, and an indicator. In an embodiment, the plunger initially protrudes into the recess. In an embodiment, when the base plate is properly placed in the recess, the indictor moves in a direction opposite to movement of the plunger via the rocker arm, which provides the indication of proper placement.

In an embodiment, a bioprocessing system is provided. The bioprocessing system includes a vessel defining an interior space for receiving a flexible bioprocessing bag, the vessel having an access door in a sidewall of the vessel and providing access to the interior space, and a tubing and component management apparatus mounted to the sidewall of the vessel and having a mounting frame for mounting of at least one consumable component of the bioprocessing system. The mounting frame is moveable vertically into and out of the interior space. In an embodiment, the mounting frame is moveable between and installation position where the mounting frame is positioned within the interior space of the vessel at a height where the mounting frame is accessible through the access door, and an operational position where the mounting frame is positioned generally above a top of the bioreactor vessel. In an embodiment, the tubing and component management apparatus includes a lifting mechanism for moving the mounting frame vertically along a centerline of the vessel. In an embodiment, the mounting frame is slidable in a direction generally perpendicular to the centerline of the vessel between a stowed position where the mounting frame is positioned within the interior space, and an access position where the mounting frame extends through an access door opening when the access door is in an open position. In an embodiment, the tubing and component management apparatus includes a locking device for selectively locking the mounting frame in the stowed position and the access position. In an embodiment, the lifting mechanism is a linear actuator. In an embodiment, the tubing and component management apparatus includes a support member mounted to a sidewall of the vessel, a boom extending from the support member generally over the vessel, and a sleeve extending downwardly from the boom along a centerline of the vessel, wherein the mounting frame includes a shaft that is received within the sleeve. In an embodiment, the lifting mechanism includes a cable extending from the mounting frame, through the sleeve and along the boom, wherein the cable is selectively extendable and retractable to selectively lower and raise the mounting frame. In an embodiment, the lifting mechanism is integrated with an internal baffle of the vessel. In an embodiment, the tubing and component management apparatus includes a guide rail mounted to an internal sidewall of the vessel and a carriage plate slidably connected to the guide rail, wherein the mounting frame is connected to the carriage plate for vertical movement along the guide rail. In an embodiment, the tubing and component management apparatus includes a baffle cover defining the internal baffle, wherein the baffle cover includes a slot through which the carriage plate extends. In an embodiment, the vessel includes a window in a sidewall of the vessel. The access door is movable between a closed position and an open position. When in the closed position, an edge of the access door defines at least a portion of a boundary of the window. In an embodiment, the mounting frame includes at least one slot, aperture or bracket for receiving the at least one consumable component. In an embodiment, the at least one consumable component is a tube, a filter or a filter heater.

In another embodiment, a method for installing components of a bioprocessing system is provided. The method includes the steps of lowering a mounting frame into a vessel through a top opening of the vessel, opening an access door in a sidewall of the vessel to access the mounting frame, mounting at least one consumable component to the mounting frame, closing the access door, and raising the mounting frame to a position adjacent to a top of the vessel. In an embodiment, the method also includes the step of moving the mounting frame in a direction generally perpendicular to a centerline of the vessel to extend the mounting frame through the access door opening. In an embodiment, the steps of lowering the mounting frame and raising the mounting frame are carried out automatically by a control unit of the bioprocessing system. In an embodiment, the method also includes actuating a lift assembly to lower or raise the mounting frame.

In yet another embodiment, a bioprocessing system is provided. The bioprocessing system includes a vessel defining an interior space for receiving a flexible bioprocessing bag, the vessel having an access door in a sidewall of the vessel and providing access to the interior space through an access door opening, and a tubing and component management apparatus mounted to the sidewall of the vessel and having a mounting frame for mounting of at least one consumable component of the bioprocessing system. The mounting frame is moveable between and installation position where the mounting frame is positioned within the interior space of the vessel at a height where the mounting frame is accessible through the access door, and an operational position where the mounting frame is positioned generally above a top of the bioreactor vessel. The tubing and component management apparatus includes a lift mechanism for moving the mounting frame between the installation position and the operational position. In an embodiment, the mounting frame is slidable in a direction generally perpendicular to the centerline of the vessel between a stowed position where the mounting frame is positioned within the interior space, and an access position where the mounting frame extends through an access door opening when the access door is in an open position. In an embodiment, the tubing and component management apparatus is mounted to an internal side of the sidewall of the vessel. In an embodiment, the lift mechanism includes a linear actuator. In an embodiment, the vessel includes a window in a sidewall of the vessel, wherein the access door is movable between a closed position and an open position, and wherein when in the closed position, an edge of the access door defines at least a portion of a boundary of the window.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property.

This written description uses examples to disclose several embodiments of the invention, including the preferred mode, and also to enable one of ordinary skill in the art to practice the embodiments of invention, including making and using any devices or systems and performing any incorporated methods. The patentable scope of the invention is defined by the claims.

## Claims

1. A bioreactor vessel (12, 112), comprising:
a bottom (314);
a peripheral sidewall, the bottom (314) and the peripheral sidewall defining an interior space (18, 118) for receiving a flexible bioprocessing bag (20);
a recess (312) in the bottom (314) for receiving a base plate (310, 410, 510) of the flexible bioprocessing bag (20); and
a locking mechanism (300) configured to retain the base plate (310, 410, 510) in the recess (312) and to secure the base plate (310, 410, 510) to the bottom of the bioreactor vessel (12, 112); and wherein:
the locking mechanism (300) includes a latch (324, 326);
wherein the latch (324, 326, 440, 540) is moveable between an engagement position where the latch (324, 326, 440, 540) engages the base plate (310, 410, 510) when the base plate (310, 410, 510) is positioned in the recess (312) in the bottom (314) of the bioreactor vessel (12, 112) to retain the base plate (310, 410, 510) in the recess (312), and a clearance position where the base plate (310, 410, 510) can be withdrawn from the recess (312).

2. The bioreactor vessel (12, 112) of claim 1, wherein:
the latch (324, 326, 440, 540) is spring-biased toward the engagement position.

3. The bioreactor vessel (12, 112) of claim 1 or 2, wherein:
the locking mechanism (300) includes a handle (446, 546) that is operable to move the latch (324, 326, 440, 540) from the engagement position to the clearance position.

4. The bioreactor vessel (12, 112) of claim 2 or 3, wherein:
the latch (324, 326, 440, 540) includes an angled upper surface configured to translate a downward force from the base plate (310, 410, 510) into a lateral force for moving the latch (324, 326, 440, 540) to the clearance position against the spring-bias during installation of the base plate (310, 410, 510) in the recess (312).

5. The bioreactor vessel (12, 112) of any preceding claim, further comprising:
a tongue (322, 464) extending into the recess (312) opposite the locking mechanism (300), the tongue (322, 464) being configured to engage a slot (320, 420) in a rear area of the base plate (310, 410, 510).

6. The bioreactor vessel (12, 112) of any preceding claim, wherein:
the locking mechanism (300) includes at least one aperture adjacent to the recess (312) and configured to receive at least one corresponding latch (324, 326, 440, 540) of the base plate (310, 410, 510).

7. The bioreactor vessel (12, 112) of claim 6, wherein:
the at least one aperture is a pair of apertures.

8. The bioreactor vessel (12, 112) of any of the preceding claims, further comprising an indicator mechanism (550) configured to indicate if the base plate (310, 410, 510) of the flexible bioprocessing bag (20) is properly positioned within the recess (312).

9. The bioreactor vessel (12, 112) of claim 8, wherein the indicator mechanism (550) comprises:
a plunger (552) located within the bottom (314), a top portion of the plunger (552) configured to protrude into the recess (312); and
a rocker arm (554) having a first end and a second end, the first end connected to the plunger (552).

10. The bioreactor vessel (12, 112) of claim 9, wherein:
the second end (555) of the rocker arm (554) comprises an indicator (556) configured to move in response to movement of the plunger (552), and
movement of the indicator (556) provides indication that the base plate (310, 410, 510) is properly or improperly placed within the recess (312).

11. The bioreactor vessel (12, 112) of claim 9 or 10, further comprising a biasing mechanism (558) located in a cutout thereof, such that prior to attachment of the base plate (510) to the bottom (314) of the bioreactor vessel, the plunger (552) is in an extended position such that a top (553) thereof protrudes into the recess (312).

12. The bioreactor vessel (12, 112) of any of claims 9 to 11, further comprising an indicator panel that is attached to, or a portion of, a housing (547).

## Patentansprüche

1. Bioreaktorgefäß (12, 112), umfassend:
einen Boden (314);
eine umlaufende Seitenwand, wobei der Boden (314) und die umlaufende Seitenwand einen Innenraum (18, 118) zum Aufnehmen eines flexiblen Bioverarbeitungsbeutels (20) definieren;
eine Aussparung (312) in dem Boden (314) zum Aufnehmen einer Grundplatte (310, 410, 510) des flexiblen Bioverarbeitungsbeutels (20); und
einen Verriegelungsmechanismus (300), der konfiguriert ist, um die Grundplatte (310, 410, 510) in der Aussparung (312) festzuhalten und die Grundplatte (310, 410, 510) am Boden des Bioreaktorgefäßes (12, 112) zu befestigen; und wobei:
der Verriegelungsmechanismus (300) einen Riegel (324, 326) beinhaltet;
wobei der Riegel (324, 326, 440, 540) zwischen einer Eingriffsposition, in welcher der Riegel (324, 326, 440, 540) mit der Grundplatte (310, 410, 510) in Eingriff steht, wenn die Grundplatte (310, 410, 510) in der Aussparung (312) in dem Boden (314) des Bioreaktorgefäßes (12, 112) positioniert ist, um die Grundplatte (310, 410, 510) in der Aussparung (312) festzuhalten, und einer Freigabeposition beweglich ist, in der die Grundplatte (310, 410, 510) aus der Aussparung (312) herausgezogen werden kann.

2. Bioreaktorgefäß (12, 112) nach Anspruch 1, wobei:
der Riegel (324, 326, 440, 540) durch eine Feder zur Eingriffsposition hin vorgespannt ist.

3. Bioreaktorgefäß (12, 112) nach Anspruch 1 oder 2, wobei:
der Verriegelungsmechanismus (300) einen Griff (446, 546) beinhaltet, der betreibbar ist, um den Riegel (324, 326, 440, 540) aus der Eingriffsposition in die Freigabeposition zu bewegen.

4. Bioreaktorgefäß (12, 112) nach Anspruch 2 oder 3, wobei:
der Riegel (324, 326, 440, 540) eine abgewinkelte obere Oberfläche beinhaltet, die konfiguriert ist, um eine nach unten gerichtete Kraft von der Grundplatte (310, 410, 510) in eine seitliche Kraft zum Bewegen des Riegels (324, 326, 440, 540) während der Installation der Grundplatte (310, 410, 510) in der Aussparung (312) gegen die Federvorspannung in die Freigabeposition umzuwandeln.

5. Bioreaktorgefäß (12, 112) nach einem vorstehenden Anspruch, weiter umfassend:
eine Zunge (322, 464), die sich in die Aussparung (312) gegenüber dem Verriegelungsmechanismus (300) erstreckt, wobei die Zunge (322, 464) konfiguriert ist, um mit einem Schlitz (320, 420) in einem hinteren Bereich der Grundplatte (310, 410, 510) in Eingriff zu stehen.

6. Bioreaktorgefäß (12, 112) nach einem vorstehenden Anspruch, wobei:
der Verriegelungsmechanismus (300) zumindest eine Öffnung neben der Aussparung (312) beinhaltet und konfiguriert ist, um zumindest einen entsprechenden Riegel (324, 326, 440, 540) der Grundplatte (310, 410, 510) aufzunehmen.

7. Bioreaktorgefäß (12, 112) nach Anspruch 6, wobei:
die zumindest eine Öffnung ein Paar von Öffnungen ist.

8. Bioreaktorgefäß (12, 112) nach einem der vorstehenden Ansprüche, weiter einen Anzeigemechanismus (550) umfassend, der konfiguriert ist, um anzuzeigen, ob die Grundplatte (310, 410, 510) des flexiblen Bioverarbeitungsbeutels (20) ordnungsgemäß in der Aussparung (312) positioniert ist.

9. Bioreaktorgefäß (12, 112) nach Anspruch 8, wobei der Anzeigemechanismus (550) umfasst:
einen Kolben (552), der sich in dem Boden (314) befindet, wobei ein oberer Abschnitt des Kolbens (552) konfiguriert ist, um in die Aussparung (312) hineinzuragen; und
einen Schwingarm (554), der ein erstes Ende und ein zweites Ende aufweist, wobei das erste Ende mit dem Kolben (552) verbunden ist.

10. Bioreaktorgefäß (12, 112) nach Anspruch 9, wobei:
das zweite Ende (555) des Schwingarmes (554) eine Anzeige (556) umfasst, die konfiguriert ist, um sich als Reaktion auf die Bewegung des Kolbens (552) zu bewegen, und
eine Bewegung der Anzeige (556) eine Anzeige bereitstellt, dass die Grundplatte (310, 410, 510) ordnungsgemäß oder nicht ordnungsgemäß in der Aussparung (312) platziert ist.

11. Bioreaktorgefäß (12, 112) nach Anspruch 9 oder 10, das weiter einen in einem Ausschnitt davon angeordneten Vorspannmechanismus (558) umfasst, sodass sich der Kolben (552) vor der Befestigung der Grundplatte (510) am Boden (314) des Bioreaktorgefäßes in einer ausgefahrenen Position befindet, sodass eine Spitze (553) davon in die Aussparung (312) hineinragt.

12. Bioreaktorgefäß (12, 112) nach einem der Ansprüche 9 bis 11, das weiter eine Anzeigetafel umfasst, die an einem Gehäuse (547) oder an einem Abschnitt davon befestigt ist.

## Revendications

1. Cuve de bioréacteur (12, 112), comprenant :
un fond (314) ;
une paroi latérale périphérique, le fond (314) et la paroi latérale périphérique définissant un espace intérieur (18, 118) pour recevoir un sac de biotraitement souple (20) ;
un évidement (312) dans le fond (314) pour recevoir une plaque de base (310, 410, 510) du sac de biotraitement souple (20) ; et
un mécanisme de verrouillage (300) configuré pour retenir la plaque de base (310, 410, 510) dans l'évidement (312) et pour fixer la plaque de base (310, 410, 510) au fond de la cuve de bioréacteur (12, 112) ; et dans laquelle :
le mécanisme de verrouillage (300) inclut un verrou (324, 326) ;
dans laquelle le verrou (324, 326, 440, 540) peut se déplacer entre une position de mise en prise dans laquelle le verrou (324, 326, 440, 540) vient en prise avec la plaque de base (310, 410, 510) lorsque la plaque de base (310, 410, 510) est positionnée dans l'évidement (312) dans le fond (314) de la cuve de bioréacteur (12, 112) pour retenir la plaque de base (310, 410, 510) dans l'évidement (312), et une position de dégagement dans laquelle la plaque de base (310, 410, 510) peut être retirée de l'évidement (312).

2. Cuve de bioréacteur (12, 112) selon la revendication 1, dans laquelle :
le verrou (324, 326, 440, 540) est sollicité par ressort vers la position de mise en prise.

3. Cuve de bioréacteur (12, 112) selon la revendication 1 ou 2, dans laquelle :
le mécanisme de verrouillage (300) inclut une poignée (446, 546) qui peut être actionnée pour déplacer le verrou (324, 326, 440, 540) de la position de mise en prise à la position de dégagement.

4. Cuve de bioréacteur (12, 112) selon la revendication 2 ou 3, dans laquelle :
le verrou (324, 326, 440, 540) inclut une surface supérieure inclinée configurée pour traduire une force vers le bas provenant de la plaque de base (310, 410, 510) en une force latérale pour déplacer le verrou (324, 326, 440, 540) jusqu'à la position de dégagement à l'encontre de la sollicitation du ressort pendant l'installation de la plaque de base (310, 410, 510) dans l'évidement (312).

5. Cuve de bioréacteur (12, 112) selon une quelconque revendication précédente, comprenant en outre :
une languette (322, 464) s'étendant dans l'évidement (312) à l'opposé du mécanisme de verrouillage (300), la languette (322, 464) étant configurée pour venir en prise avec une fente (320, 420) dans une zone arrière de la plaque de base (310, 410, 510).

6. Cuve de bioréacteur (12, 112) selon une quelconque revendication précédente, dans laquelle :
le mécanisme de verrouillage (300) inclut au moins un orifice adjacent à l'évidement (312) et configuré pour recevoir au moins un verrou correspondant (324, 326, 440, 540) de la plaque de base (310, 410, 510).

7. Cuve de bioréacteur (12, 112) selon la revendication 6, dans laquelle :
le au moins un orifice est une paire d'orifices.

8. Cuve de bioréacteur (12, 112) selon une quelconque revendication précédente, comprenant en outre un mécanisme indicateur (550) configuré pour indiquer si la plaque de base (310, 410, 510) du sac de biotraitement souple (20) est positionnée correctement à l'intérieur du évidement (312).

9. Cuve de bioréacteur (12, 112) selon la revendication 8, dans lequel le mécanisme indicateur (550) comprend :
un piston (552) situé à l'intérieur du fond (314), une partie supérieure du piston (552) étant configurée pour faire saillie dans l'évidement (312) ; et
un bras oscillant (554) présentant une première extrémité et une seconde extrémité, la première extrémité étant reliée au piston (552).

10. Cuve de bioréacteur (12, 112) selon la revendication 9, dans laquelle :
la seconde extrémité (555) du bras oscillant (554) comprend un indicateur (556) configuré pour se déplacer en réponse à un mouvement du piston (552), et
le déplacement de l'indicateur (556) fournit une indication que la plaque de base (310, 410, 510) est placée correctement ou incorrectement dans l'évidement (312).

11. Cuve de bioréacteur (12, 112) selon la revendication 9 ou 10, comprenant en outre un mécanisme de sollicitation (558) situé dans une découpe de celle-ci, de telle sorte qu'avant la fixation de la plaque de base (510) au fond (314) de la cuve de bioréacteur, le piston (552) soit dans une position étendue de telle sorte qu'une partie supérieure (553) de celui-ci fasse saillie dans l'évidement (312).

12. Cuve de bioréacteur (12, 112) selon l'une quelconque des revendications 9 à 11, comprenant en outre un panneau indicateur qui est fixé à un boîtier (547), ou à une partie de celui-ci.
